# EUROPEAN PATENT APPLICATION

(11) **EP 3 964 201 A1**
(43) Date of publication of application: **09.03.2022**
(21) Application number: 21787288.6
(22) Date of filing: 05.07.2021
(51) Int. Cl.: A61K 9/22, A61K 9/28, A61K 31/4015, A61P 25/08

(54) **BRIVARACETAM PHARMACEUTICAL COMPOSITION, PREPARATION METHOD THEREFOR AND APPLICATION THEREOF**

(30) Priority: 09.07.2020 CN 202010655757
(71) Applicant: Shanghai Bocimed Pharmaceutical Co., Ltd., Shanghai 201210 (CN); Shanghai Bocimed Pharmaceutical Research Co., Ltd., Shanghai 201208 (CN)
(72) Inventor: GUO, Zhen, Shanghai 201210 (CN); CHEN, Lina, Shanghai 201210 (CN); WEN, Yifeng, Shanghai 201210 (CN); WANG, Tingting, Shanghai 201210 (CN); YING, Shuhuan, Shanghai 201210 (CN)
(74) Representative: Gille Hrabal Partnerschaftsgesellschaft mbB Patentanwälte
(86) International application number: PCT/CN2021/104586
(87) International publication number: WO 2022/007758

(57) **Abstract**

The present disclosure provides a brivaracetam pharmaceutical composition, a preparation method therefor and use thereof. The pharmaceutical composition comprises an active pharmaceutical ingredient, a matrix forming agent, and a swelling agent. The brivaracetam pharmaceutical composition disclosed herein has a sustained release effect and has a more flat release curve compared with a common gel skeleton sustained-release preparation, and thus achieves the purposes of reducing the release rate of the medicament, controlling the *in vivo* effective dosage of the medicament, stabilizing the blood concentration, reducing toxic and side effects and reducing the times of daily administration.

## Description

The present application claims priority to Patent Application No. 202010655757.X filed to China National Intellectual Property Administration on Jul. 9, 2020 entitled "BRIVARACETAM PHARMACEUTICAL COMPOSITION, PREPARATION METHOD THEREFOR AND USE THEREOF", which is incorporated herein by reference in its entirety.

### TECHNICAL FIELD

The present disclosure belongs to the field of pharmaceutical compositions, and relates to a brivaracetam pharmaceutical composition, a preparation method therefor and use thereof.

### BACKGROUND

Brivaracetam, as an antiepileptic drug, is clinically used as a single drug therapy for partial seizure type epilepsy patients aged 4 or more.

Brivaracetam has a chemical name of (2*S*)-2-[(4*R*)-2-oxo-4-propyltetrahydron-1*H*-pyrro-1-yl]butanamide and a molecular formula of C₁₁H₂₀N₂O₂, and the structural formula thereof is as follows:

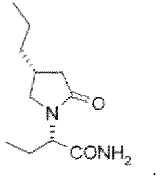

Brivaracetam, a BCS I type medicament in a form of white or off-white crystal powder, is easy to dissolve in water (0.7 g/mL), a hydrochloric acid solution with a pH of 1.2 (0.85 g/mL), a buffer solution with a pH of 4.5 (0.85 g/mL) and a buffer solution with a pH of 7.4 (0.84 g/mL), and has a short terminal half-life period (9 h), and thus the effective plasma concentration of the medicament in a body is maintained for a short time. The existing commercially-available preparations comprise a brivaracetam tablet, a brivaracetam oral liquid and a brivaracetam injection, wherein the brivaracetam tablet has a plurality of specifications such as 10 mg, 25 mg, 50 mg, 75 mg and 100 mg, and both the brivaracetam oral liquid and the brivaracetam injection have a specification of 10 mg/mL.

The administration method for the commercially-available brivaracetam tablet comprises: administering the medicament at a recommended initial dosage of 50 mg twice a day, and adjusting the dosage to 25 mg twice a day or 100 mg twice a day depending on the tolerance and the response to treatment of an individual patient. That is, the *in vivo* dosage cannot be effectively controlled in clinical use due to the fast release of the commercially-available tablet. In addition, in this administration method, the dosage of the medicament needs to be adjusted according to the disease condition of the patient after the medicament is taken, which is not beneficial for patients to take the medicament on their own.

In the case where most of the brivaracetam tablets prepared in the prior art are immediate release tablets, a patent (grant announcement No.: CN102046153B) of UCB company discloses a novel pharmaceutical composition comprising brivaracetam, wherein the novel pharmaceutical composition is capable of controlling the release rate of the drug and provides a therapeutic effect for at least 16 h during administration. However, the release rate of the pharmaceutical composition prepared by the method is relatively high, and products prepared by this process have not seen on the market yet.

Therefore, there is an urgent need to develop a sustained-release tablet to achieve the purposes of reducing the drug release rate, controlling the *in vivo* effective dosage of the drug, and reducing the times of daily administration. The compliance of a patient can be improved by once-daily administration, and by reducing the highest content of the drug in blood, the time of the drug *in vivo* can be prolonged, the *in vivo* effective dosage of the drug can be controlled, and the toxic and side effects of the drug can be reduced, thus achieving the effective therapeutic effect.

### SUMMARY

In order to improve the above technical problem, the present disclosure provides a brivaracetam pharmaceutical composition which is a 24-hour sustained-release drug and has a dissolution simultaneously meeting the following three characteristics:
A) not more than 40% of an active pharmaceutical ingredient is dissolved out within 1 h;
B) 20%-70% of the active pharmaceutical ingredient is dissolved out within 6 h; and
C) not less than 65% of the active pharmaceutical ingredient is dissolved out within 24 h;
the active pharmaceutical ingredient is selected from one, two or more of brivaracetam, a pharmaceutically acceptable complex of the brivaracetam, a pharmaceutically acceptable salt of the brivaracetam, a pharmaceutically acceptable solvate of the brivaracetam and a pharmaceutically acceptable hydrate of the brivaracetam

Preferably, the brivaracetam pharmaceutical composition has a dissolution simultaneously meeting the following three characteristics:
A) not more than 40% of the brivaracetam or the pharmaceutically acceptable salt thereof is dissolved out within 1 h;
B) 20%-70% of the brivaracetam or the pharmaceutically acceptable salt thereof is dissolved out within 6 h; and
C) not less than 65% of the brivaracetam or the pharmaceutically acceptable salt thereof is dissolved out within 24 h.

Preferably, the brivaracetam pharmaceutical composition is a 24-hour sustained-release drug and has a dissolution simultaneously meeting the following three characteristics:
A) not more than 35% of the active pharmaceutical ingredient is dissolved out within 1 h; preferably, not more than 35% of the brivaracetam or the pharmaceutically acceptable salt thereof is dissolved out within 1 h;
B) 30%-70% of the active pharmaceutical ingredient is dissolved out within 6 h; preferably, 30%-70% of the brivaracetam or the pharmaceutically acceptable salt thereof is dissolved out within 6 h; and
C) not less than 75% of the active pharmaceutical ingredient is dissolved out within 24 h; preferably, not less than 75% of the brivaracetam or the pharmaceutically acceptable salt thereof is dissolved out within 24 h.

Also preferably, the brivaracetam pharmaceutical composition has a dissolution simultaneously meeting the following three characteristics:
A) not more than 30% of the active pharmaceutical ingredient is dissolved out within 1 h; preferably, not more than 30% of the brivaracetam or the pharmaceutically acceptable salt thereof is dissolved out within 1 h;
B) 30%-65% of the active pharmaceutical ingredient is dissolved out within 6 h; preferably, 30%-65% of the brivaracetam or the pharmaceutically acceptable salt thereof is dissolved out within 6 h; and
C) not less than 75% of the active pharmaceutical ingredient is dissolved out within 24 h; preferably, not less than 75% of the brivaracetam or the pharmaceutically acceptable salt thereof is dissolved out within 24 h.

More preferably, the brivaracetam pharmaceutical composition has a dissolution simultaneously meeting the following three characteristics:
A) not more than 30% of the active pharmaceutical ingredient is dissolved out within 1 h; preferably, not more than 30% of the brivaracetam or the pharmaceutically acceptable salt thereof is dissolved out within 1 h;
B) 30%-65% of the active pharmaceutical ingredient is dissolved out within 6 h; preferably, 30%-65% of the brivaracetam or the pharmaceutically acceptable salt thereof is dissolved out within 6 h; and
C) not less than 80% of the active pharmaceutical ingredient is dissolved out within 24 h; preferably, not less than 80% of the brivaracetam or the pharmaceutically acceptable salt thereof is dissolved out within 24 h.

The "dissolution" refers to a cumulative dissolution rate of the active pharmaceutical ingredient (such as the brivaracetam or the pharmaceutically acceptable salt thereof); furthermore, the cumulative dissolution rate is measured in an acetate buffer at a pH of 4.5. Those skilled in the art will understand that the dissolution rate of the brivaracetam or the pharmaceutically acceptable salt thereof gradually increases over time.

The present disclosure provides a brivaracetam pharmaceutical composition, which comprises an active pharmaceutical ingredient, a matrix forming agent and a swelling agent;
the active pharmaceutical ingredient is selected from one, two or more of the following substances: brivaracetam, a pharmaceutically acceptable complex of the brivaracetam, a pharmaceutically acceptable salt of the brivaracetam, a pharmaceutically acceptable solvate of the brivaracetam, and a pharmaceutically acceptable hydrate of the brivaracetam;
the matrix forming agent refers to a substance capable of providing structural integrity and facilitating the control or prolonging of the release rate of the medicament; for example, the matrix forming agent is selected from one, two or more of the following substances: polyvinyl acetate (PVAc), polyvinylpyrrolidone (PVP), carbomer, polysaccharide, polyacrylic resin, polyvinyl acetate povidone mixture (Kollidon^{®}SR, hereinafter referred to as KSR), and polyvinyl alcohol;
the swelling agent refers to a substance capable of absorbing water from gastric fluid to enable the expansion of the size of the solid preparation and affecting the release rate of the medicament by generating channels or by forming hydrophilic colloids; for example, the swelling agent is selected from one, two or more of the following substances: crospolyvinylpyrrolidone (PVPP), polyethylene oxide (PEO), croscarmellose sodium, sodium carboxymethyl starch, low-substituted hydroxypropylcellulose, carboxymethylcellulose calcium, carboxymethylcellulose, alginate, calcium salt, and polacrilin potassium.

Furthermore, the alginate may be a salt formed by an alginic acid and metal ions (such as sodium ions and potassium ions), such as sodium alginate and/or potassium alginate; furthermore, the calcium salt may be a salt formed by an inorganic acid and calcium ions, or a salt formed by an organic acid and calcium ions; for example, the calcium salt is selected from one, two or three of calcium chloride, dicalcium phosphate and dicalcium phosphate dihydrate.

According to an embodiment of the present disclosure, the active pharmaceutical ingredient is preferably brivaracetam. For example, a weight percentage of the active pharmaceutical ingredient is 2.00%-50.00%, more preferably 3.00%-20.00%, such as 9.10%, 11.10%, 9.09%, 4.55% or 18.18%; the weight percentage refers to the percentage of the weight of the active pharmaceutical ingredient in the total weight of the brivaracetam pharmaceutical composition.

According to an embodiment of the present disclosure, a weight percentage of the matrix forming agent is 5.00%-60.00%, more preferably 30.00%-50.00%, such as 49.10%, 50.00%, 38.00%, 36.09%, 36.36%, 38.91%, or 36.54%; the weight percentage refers to the percentage of the weight of the matrix forming agent in the total weight of the brivaracetam pharmaceutical composition. The KSR may be an 80/19 (w/w) mixture comprising PVAc and PVP, available from BASF under the trade name of KOLLIDON^{@}SR.

For example, the polysaccharide is selected from one, two or more of the following polysaccharides: xanthan gum, inulin, guar gum, chitosan, carob gum, carrageenan and a cellulose derivative.

For example, the cellulose derivative is selected from one, two or more of the following substances: ionic cellulose polymer and nonionic cellulose polymer.

Preferably, the ionic cellulose polymer is selected from one, two or more of the following substances: carboxymethylcellulose (CMC), carboxymethylcellulose sodium, carboxymethylcellulose calcium, carboxyethylcellulose (CEC), carboxymethylethylcellulose (CMEC), hydroxyethyl methylcellulose acetate phthalate, hydroxyethyl methylcellulose acetate succinate, hydroxypropylcellulose acetate phthalate (HPCAP), hydroxypropylcellulose acetate succinate (HPCAS), and hydroxypropyl methylcellulose acetate phthalate (HPMCAP).

Preferably, the nonionic cellulose polymer is selected from one, two or more of the following substances: methylcellulose (MC), ethylcellulose (EC), hydroxyethylcellulose (HEC), hydroxypropylcellulose (HPC), hydroxypropyl methylcellulose (HPMC), hydroxypropylmethylcellulose acetate, hydroxyethyl methylcellulose, hydroxyethylcellulose acetate and hydroxyethyl ethylcellulose.

According to an embodiment of the present disclosure, the swelling agent is soluble or insoluble in water. Preferably, the swelling agent may be selected from one, two or more of crospolyvinylpyrrolidone (PVPP), polyethylene oxide (PEO), croscarmellose sodium, sodium carboxymethyl starch, low-substituted hydroxypropylcellulose, carboxymethylcellulose calcium, carboxymethylcellulose and polacrilin potassium.

Furthermore, a weight percentage of the swelling agent is 5.00%-60.00%, more preferably 20.00%-50.00%, for example 40.80%, 37.90%, 31.00%, 35.46%, 36.36%, 38.17% or 34.55%; the weight percentage refers to the percentage of the swelling agent in the total weight of the brivaracetam pharmaceutical composition.

According to an embodiment of the present disclosure, the brivaracetam pharmaceutical composition may further comprise a lubricant and/or a diluent.

According to an embodiment of the present disclosure, the lubricant is a substance that facilitates the performance of the processing steps such as component mixing and tableting, and may be selected from one, two or more of talc, stearic acid, metal stearate, stearate, glyceryl behenate, sodium lauryl sulfate, hydrogenated vegetable oil, mineral oil, poloxamer (copolymer of ethylene oxide and propylene oxide), polyethylene glycol and sodium chloride. Furthermore, the metal stearate is selected from one, two or three of calcium stearate, magnesium stearate and zinc stearate, preferably magnesium stearate. Furthermore, the stearate is selected from one, two or more of polyoxyethylene stearate, glyceryl monostearate, glyceryl palmitostearate, and the like.

According to an embodiment of the present disclosure, the diluent refers to a substance capable of improving the flowability, enhancing the compressive strength or hardness, reducing the friability and the like of the pharmaceutical composition during the component mixing and tableting. For example, the diluent is selected from one, two or more of dextrose, lactose monohydrate, anhydrous lactose, sucrose, mannitol, xylitol, sorbitol, microcrystalline cellulose, starch, pregelatinized starch, dicalcium phosphate dihydrate, anhydrous dicalcium phosphate, cyclodextrin and cyclodextrin derivative. Furthermore, the cyclodextrin is selected from one, two or more of α-cyclodextrin, β-cyclodextrin and gamma-cyclodextrin; the cyclodextrin derivative is selected from one, two or more of cyclodextrin glucose derivative, cyclodextrin hydroxypropyl derivative, cyclodextrin methyl derivative, cyclodextrin ethyl derivative, cyclodextrin acetyl derivative, cyclodextrin sulfobutyl derivative, ionic cyclodextrin derivative and the like.

According to an embodiment of the present disclosure, a weight percentage of the lubricant may be 0%-3.00%, more preferably 0.50%-2.00%, for example, 1.00%, wherein the weight percentage refers to the percentage of the weight of the lubricant in the total weight of the brivaracetam pharmaceutical composition.

According to an embodiment of the present disclosure, a weight percentage of the diluent may be 0%-30.00%, more preferably 0%-20.00%, for example, 18.90%, 18.36%, 17.18%, 17.36% or 9.73%, wherein the weight percentage refers to the percentage of the weight of the diluent in the total weight of the brivaracetam pharmaceutical composition.

Preferably, the brivaracetam pharmaceutical composition has the dissolution characteristics as shown above.

The brivaracetam pharmaceutical composition provided herein is stable and is suitable for being orally taken once a day. The pharmaceutical composition, when administered in a solid dosage form, has a longer retention time in a stomach than an immediate release preparation. The pharmaceutical composition, when retained in the stomach, can continuously release brivaracetam.

According to a preferred embodiment of the present disclosure, the brivaracetam pharmaceutical composition consists of an active pharmaceutical ingredient, a matrix forming agent and a swelling agent;
the active pharmaceutical ingredient is selected from one, two or more of brivaracetam, a pharmaceutically acceptable complex of the brivaracetam, a pharmaceutically acceptable salt of the brivaracetam, a pharmaceutically acceptable solvate of the brivaracetam and a pharmaceutically acceptable hydrate of the brivaracetam;
the matrix forming agent is selected from one, two or more of polyvinyl acetate (PVAc), polyvinylpyrrolidone (PVP), KSR, carbomer and polysaccharide;
the swelling agent is selected from one, two or more of crospolyvinylpyrrolidone (PVPP), polyethylene oxide (PEO), alginate and calcium salt.

According to a preferred embodiment of the present disclosure, the brivaracetam pharmaceutical composition may consist of the following components: brivaracetam, KSR, crospolyvinylpyrrolidone (PVPP), polyethylene oxide (PEO), carbomer and magnesium stearate.

According to a preferred embodiment of the present disclosure, the brivaracetam pharmaceutical composition may consist of the following components: brivaracetam, KSR, crospolyvinylpyrrolidone (PVPP), polyethylene oxide (PEO), carbomer, lactose and magnesium stearate.

According to a preferred embodiment of the present disclosure, the brivaracetam pharmaceutical composition may consist of the following components: brivaracetam, KSR, crospolyvinylpyrrolidone (PVPP), polyethylene oxide (PEO), sodium carboxymethyl starch, hydroxypropyl methylcellulose, pregelatinized starch, carbomer, microcrystalline cellulose and magnesium stearate.

According to a preferred embodiment of the present disclosure, the brivaracetam pharmaceutical composition may consist of the following components: brivaracetam, KSR, crospolyvinylpyrrolidone (PVPP), sodium alginate, calcium chloride, sodium carboxymethyl starch, hydroxypropyl methylcellulose, pregelatinized starch, microcrystalline cellulose and magnesium stearate.

According to a preferred embodiment of the present disclosure, the brivaracetam pharmaceutical composition consists of the following components: brivaracetam, KSR, crospolyvinylpyrrolidone (PVPP), sodium carboxymethyl starch, hydroxypropyl methylcellulose, pregelatinized starch, carbomer, microcrystalline cellulose and magnesium stearate.

According to a preferred embodiment of the present disclosure, the brivaracetam pharmaceutical composition consists of the following components: brivaracetam, KSR, crospolyvinylpyrrolidone (PVPP), polyethylene oxide (PEO), sodium carboxymethyl starch, hydroxypropyl methylcellulose, pregelatinized starch, carbomer and magnesium stearate.

According to an exemplary embodiment of the present disclosure, the brivaracetam pharmaceutical composition is selected from any one of the following formulations in percentage:
formulation I: 9.1% of brivaracetam, 40.0% of KSR (i.e., KOLLIDON@SR), 20.0% of crospolyvinylpyrrolidone (PVPP), 20.8% of polyethylene oxide (PEO), 9.1% of carbomer, and 1.0% of magnesium stearate; the percentage refers to a weight percentage, wherein the weight percentage refers to the percentage of the weight of each component in the total weight of the brivaracetam pharmaceutical composition;
formulation II: 11.1% of brivaracetam, 40.0% of KSR (i.e., KOLLIDON@SR), 20.0% of crospolyvinylpyrrolidone (PVPP), 17.9% of polyethylene oxide (PEO), 10.0% of carbomer, and 1.0% of magnesium stearate; the percentage refers to a weight percentage, wherein the weight percentage refers to the percentage of the weight of each component in the total weight of the brivaracetam pharmaceutical composition;
formulation III: 11.1% of brivaracetam, 30.0% of KSR (i.e., KOLLIDON@SR), 15.0% of crospolyvinylpyrrolidone (PVPP), 16.0% of polyethylene oxide (PEO), 8.0% of carbomer, 18.9% of lactose, and 1.0% of magnesium stearate; the percentage refers to a weight percentage, wherein the weight percentage refers to the percentage of the weight of each component in the total weight of the brivaracetam pharmaceutical composition;
formulation IV: 11.1% of brivaracetam, 30.0% of KSR (i.e., KOLLIDON@SR), 15.0% of crospolyvinylpyrrolidone (PVPP), 16.0% of polyethylene oxide (PEO), 8.0% of carbomer, 8.9% of lactose, 10.0% of hydroxypropyl-β-cyclodextrin, and 1.0% of magnesium stearate; the percentage refers to a weight percentage, wherein the weight percentage refers to the percentage of the weight of each component in the total weight of the brivaracetam pharmaceutical composition;
Formulation V: 9.09% of brivaracetam, 29.09% of KSR (i.e., KOLLIDON@SR), 15.46% of crospolyvinylpyrrolidone (PVPP), 14.55% of polyethylene oxide (PEO), 5.45% of sodium carboxymethyl starch, 5.00% of hydroxypropyl methylcellulose, 10.00% of pregelatinized starch, 2.0% of carbomer, 8.36% of microcrystalline cellulose, and 1.0% of magnesium stearate; the percentage refers to a weight percentage, wherein the weight percentage refers to the percentage of the weight of each component in the total weight of the brivaracetam pharmaceutical composition;
formulation VI: 9.09% of brivaracetam, 29.09% of KSR (i.e., KOLLIDON@SR), 15.46% of crospolyvinylpyrrolidone (PVPP), 10.00% of sodium alginate, 4.55% of calcium chloride, 6.36% of sodium carboxymethyl starch, 7.27% of hydroxypropyl methylcellulose, 7.27% of pregelatinized starch, 9.91% of microcrystalline cellulose, and 1.0% of magnesium stearate; the percentage refers to a weight percentage, wherein the weight percentage refers to the percentage of the weight of each component in the total weight of the brivaracetam pharmaceutical composition;
formulation VII: 4.55% of brivaracetam, 30.0% of KSR (i.e., KOLLIDON@SR), 16.36% of crospolyvinylpyrrolidone (PVPP), 15.46% of polyethylene oxide (PEO), 6.36% of sodium carboxymethyl starch, 5.91% of hydroxypropyl methylcellulose, 11.09% of pregelatinized starch, 3.0% of carbomer, 6.27% of microcrystalline cellulose, and 1.0% of magnesium stearate; the percentage refers to a weight percentage, wherein the weight percentage refers to the percentage of the weight of each component in the total weight of the brivaracetam pharmaceutical composition; and
formulation VIII: 18.18% of brivaracetam, 29.09% of KSR (i.e., KOLLIDON@SR), 14.55% of crospolyvinylpyrrolidone (PVPP), 14.55% of polyethylene oxide (PEO), 5.45% of sodium carboxymethyl starch, 5.45% of hydroxypropyl methylcellulose, 9.73% of pregelatinized starch, 2.0% of carbomer, and 1.0% of magnesium stearate; the percentage refers to a weight percentage, wherein the weight percentage refers to the percentage of the weight of each component in the total weight of the brivaracetam pharmaceutical composition.

The present disclosure further provides a brivaracetam sustained-release tablet, which comprises the above pharmaceutical composition.

Preferably, the brivaracetam sustained-release tablet consists of a tablet core and a coating, wherein the tablet core comprises the above pharmaceutical composition.

Preferably, the tablet core consists of the following components in percentage by weight: 2.00%-50.00% of the brivaracetam, 5.00%-60.00% of the matrix forming agent, 5.00%-60.00% of the swelling agent, 0%-3.00% of the lubricant and 0%-30.00% of the diluent, wherein the percentage refers to the percentage of the weight of each component in the total weight of the brivaracetam sustained-release tablet.

Preferably, the tablet core consists of the following components in percentage by weight: 5.00%-50.00% of the brivaracetam, 5.00%-60.00% of the matrix forming agent, 5.00%-60.00% of the swelling agent, 0%-3.00% of the lubricant and 0%-30.00% of the diluent, wherein the weight percentage refers to the percentage of the weight of each component in the total weight of the brivaracetam sustained-release tablet.

Preferably, the tablet core consists of the following components in percentage by weight: 3.00%-20.00% of the brivaracetam, 30.00%-50.00% of the matrix forming agent, 20.00%-50.00% of the swelling agent, 0.50%-2.00% of the lubricant and 0%-20.00% of the diluent, wherein the percentage refers to the percentage of the weight of each component in the total weight of the brivaracetam sustained-release tablet. Preferably, the tablet core of the brivaracetam sustained-release tablet consists of the following components in percentage by weight: 6.00%-15.00% of the brivaracetam, 30.00%-50.00% of the matrix forming agent, 30.00%-50.00% of the swelling agent, 0.50%-2.00% of the lubricant and 0%-20.00% of the diluent, wherein the weight percentage refers to the percentage of the weight of each component in the total weight of the brivaracetam sustained-release tablet.

According to an exemplary embodiment of the present disclosure, a tablet core of the brivaracetam sustained-release tablet is selected from any one of the following formulations in percentage:
prescription I: 9.1% of brivaracetam, 40.0% of KSR (i.e., KOLLIDON@SR), 20.0% of crospolyvinylpyrrolidone (PVPP), 20.8% of polyethylene oxide (PEO), 9.1% of carbomer, and 1.0% of magnesium stearate; the percentage refers to a weight percentage, wherein the weight percentage refers to the percentage of the weight of each component in the total weight of the tablet core of the brivaracetam sustained-release tablet;
prescription II: 11.1% of brivaracetam, 40.0% of KSR (i.e., KOLLIDON@SR), 20.0% of crospolyvinylpyrrolidone (PVPP), 17.9% of polyethylene oxide (PEO), 10.0% of carbomer, and 1.0% of magnesium stearate; the percentage refers to a weight percentage, wherein the weight percentage refers to the percentage of the weight of each component in the total weight of the tablet core of the brivaracetam sustained-release tablet;
prescription III: 11.1% of brivaracetam, 30.0% of KSR (i.e., KOLLIDON@SR), 15.0% of crospolyvinylpyrrolidone (PVPP), 16.0% of polyethylene oxide (PEO), 8.0% of carbomer, 18.9% of lactose, and 1.0% of magnesium stearate; the percentage refers to a weight percentage, wherein the weight percentage refers to the percentage of the weight of each component in the total weight of the tablet core of the brivaracetam sustained-release tablet;
prescription IV: 11.1% of brivaracetam, 30.0% of KSR (i.e., KOLLIDON@SR), 15.0% of crospolyvinylpyrrolidone (PVPP), 16.0% of polyethylene oxide (PEO), 8.0% of carbomer, 8.9% of lactose, 10.0% of hydroxypropyl-β-cyclodextrin, and 1.0% of magnesium stearate; the percentage refers to a weight percentage, wherein the weight percentage refers to the percentage of the weight of each component in the total weight of the tablet core of the brivaracetam sustained-release tablet;
prescription V: 9.09% of brivaracetam, 29.09% of KSR (i.e., KOLLIDON@SR), 15.46% of crospolyvinylpyrrolidone (PVPP), 14.55% of polyethylene oxide (PEO), 5.45% of sodium carboxymethyl starch, 5.00% of hydroxypropyl methylcellulose, 10.00% of pregelatinized starch, 2.00% of carbomer, 8.36% of microcrystalline cellulose, and 1.00% of magnesium stearate; the percentage refers to a weight percentage, wherein the weight percentage refers to the percentage of the weight of each component in the total weight of the tablet core of the brivaracetam sustained-release tablet;
prescription VI: 9.09% of brivaracetam, 29.09% of KSR (i.e., KOLLIDON@SR), 15.46% of crospolyvinylpyrrolidone (PVPP), 10.00% of sodium alginate, 4.55% of calcium chloride, 6.36% of sodium carboxymethyl starch, 7.27% of hydroxypropyl methylcellulose, 7.27% of pregelatinized starch, 9.91% of microcrystalline cellulose, and 1.00% of magnesium stearate; the percentage refers to a weight percentage, wherein the weight percentage refers to the percentage of the weight of each component in the total weight of the tablet core of the brivaracetam sustained-release tablet;
prescription VII: 4.55% of brivaracetam, 30.0% of KSR (i.e., KOLLIDON@SR), 16.36% of crospolyvinylpyrrolidone (PVPP), 15.46% of polyethylene oxide (PEO), 6.36% of sodium carboxymethyl starch, 5.91% of hydroxypropyl methylcellulose, 11.09% of pregelatinized starch, 3.00% of carbomer, 6.27% of microcrystalline cellulose, and 1.00% of magnesium stearate; the percentage refers to a weight percentage, wherein the weight percentage refers to the percentage of the weight of each component in the total weight of the tablet core of the brivaracetam sustained-release tablet; and
prescription VIII: 18.18% of brivaracetam, 29.09% of KSR (i.e., KOLLIDON@SR), 14.55% of crospolyvinylpyrrolidone (PVPP), 14.55% of polyethylene oxide (PEO), 5.45% of sodium carboxymethyl starch, 5.45% of hydroxypropyl methylcellulose, 9.73% of pregelatinized starch, 2.00% of carbomer, and 1.00% of magnesium stearate; the percentage refers to a weight percentage, wherein the weight percentage refers to the percentage of the weight of each component in the total weight of the tablet core of the brivaracetam sustained-release tablet.

The present disclosure further provides a preparation method for the brivaracetam pharmaceutical composition or the brivaracetam sustained-release tablet, wherein the preparation method may be direct tableting, dry granulation, wet granulation or melt granulation.

For example, the preparation method for the brivaracetam pharmaceutical composition or the brivaracetam sustained-release tablet is selected from any one of the following methods:
method I: the components (such as the active pharmaceutical ingredient, the matrix forming agent and the swelling agent) of the pharmaceutical composition are directly mixed and tabletted to obtain a tablet, namely the brivaracetam sustained-release tablet; and
method II: some components (comprising the active pharmaceutical ingredient, the matrix forming agent, the swelling agent, the diluent and the lubricant) are granulated by a fluidized bed or a dry granulation method to granulate the active pharmaceutical ingredient (API) and each component, and then the granulated components are mixed with the rest components (comprising the matrix forming agent, the swelling agent, the diluent and the lubricant) and tabletted to obtain a tablet, namely the brivaracetam sustained-release tablet.

According to an embodiment of the present disclosure, the tablet may be further coated to obtain a coated tablet, namely the brivaracetam sustained-release tablet.

The present disclosure further provides use of the brivaracetam pharmaceutical composition or the brivaracetam sustained-release tablet for preparing a medicament. Furthermore, the medicament is used for the treatment and/or prevention of the following diseases: epilepsy, Parkinson, dyskinesia, migraine, tremor, essential tremor, bipolar disorder, chronic disease, neuropathic pain or bronchial, asthma or allergic diseases, etc.

The present disclosure further provides a method for treating a disorder of a patient responsive to brivaracetam, the method comprising orally administering to the patient the brivaracetam pharmaceutical composition or the brivaracetam sustained-release tablet described above once a day.

When the brivaracetam pharmaceutical composition or the brivaracetam sustained-release tablet is taken integrally and enters the stomach of the patient, it can quickly float in gastric fluid and slowly expand or swell. The preparation, when expanding to a certain size, can prevent itself from leaving the stomach through a pylorus. Since an adult has a pylorus diameter of about 12 mm, the size of the expanded preparation is higher than 13 mm, for example, 13 mm-20 mm. The brivaracetam sustained-release tablet may have any shape, such as a circle, an oval, an irregular shape and a polygon.

In the present disclosure, the "pharmaceutically acceptable" refers to salts, complexes, solvates, hydrates of drugs (e.g., brivaracetam) which are suitable for use in contact with the tissues of patients within the scope of normal medical judgment without undue toxicity, irritation, allergic response and the like, have a reasonable advantage to disadvantage ratio, and are useful for their intended use.

The "solvate" refers to a molecular complex comprising drugs (e.g., brivaracetam) and a stoichiometric or non-stoichiometric amount of one or more pharmaceutically acceptable solvent molecules (e.g., ethanol). The complex, formed by tight bonding of the solvent and the drug, has a well-defined stoichiometry, independent of humidity. However, when the solvent has weak binding properties (as in channel solvates and hygroscopic compounds), the content of the solvent depends on humidity and drying conditions; in this case, the complex is generally non-stoichiometric.

The "hydrate" refers to a solvate comprising drugs (e.g., brivaracetam) and a stoichiometric or non-stoichiometric amount of water.

In the present disclosure, the polyvinylpyrrolidone (PVP), also known as povidone or povidonum, is a homopolymer of 1-vinyl-pyrrolidin-2-one and typically has a molecular weight M_{w} of about 1 × 10³ to about 1 × 10⁷, about 2.5 × 10³ to about 3 × 10⁶ or about 1 × 10⁴ to about 1 × 10⁵. The polyvinylpyrrolidone may either be available from BASF under the trade name of KOLLIDON or from ISP under the trade name of PLASDONE^{®}.

In the present disclosure, the polyvinyl acetate (PVAc) is a homopolymer of vinyl acetate and typically has a molecular weight M_{w} of about 1 × 10⁵ to about 1 × 10⁶.

KSR may be available from BSAF under the trade name of KOLLIDON^{®}SR, known as an 80/19 (w/w) mixture of PVAc and PVP.

In the present disclosure, the polyethylene oxide (PEO) is also known as polyoxirane and polyoxyethylene. PEO is a homopolymer of ethylene oxide and typically has a molecular weight M_{w} of about 1 × 10⁵ to about 1 × 10⁷ or about 1 × 10⁶ to about 1 × 10⁷. PEO is available in various grades based on molecular weight, commercially available from UnionCarbide under the trade name of POLYOX^{®}.

The above preferred conditions may be combined arbitrarily to obtain preferred embodiments of the present disclosure without departing from the general knowledge in the art.

### Advantageous effects

The present disclosure significantly overcomes the disadvantages of the prior art that the release rate of the brivaracetam preparation is too fast, the administration times are multiple, the dosage of the medicament is not easy to control, the toxic and side effects are great, the treatment effect is limited, and the like. The brivaracetam pharmaceutical composition or the brivaracetam sustained-release tablet provided herein has a sustained-release effect, has a more flat release curve compared with an existing gel skeleton sustained-release preparation, effectively reduces the drug release rate, and controls the *in vivo* effective dosage of the medicament, thereby realizing the effects of more stable blood concentration, reduced toxic and side effects, reduced times of daily administration, and improved drug compliance. In addition, the brivaracetam pharmaceutical composition or the brivaracetam sustained-release tablet provided herein has excellent stability.

### BRIEF DESCRIPTION OF THE DRAWINGS

FIG. 1 is a curve diagram of dissolutions of tablets A, B, C, D, E, F, G and H prepared according to the present disclosure and a sustained-release tablet prepared according to patent CN102046153B; and
FIG. 2 is a time-concentration curve diagram of a test sample group and a control group of original study during the PK study in Beagle dogs.

### DETAILED DESCRIPTION

The technical scheme of the present disclosure will be further illustrated in detail with reference to the following specific examples. It should be understood that the following examples are merely exemplary illustration and explanation of the present disclosure, and should not be construed as limiting the protection scope of the present disclosure. All techniques implemented based on the aforementioned contents of the present disclosure are encompassed within the protection scope of the present disclosure.

Unless otherwise specified, the raw materials and reagents used in the following examples are all commercially available products or can be prepared by known methods.

Raw and auxiliary materials in Example 1 comprise as follows:
brivaracetam: purchased from Ruyuan Dongyangguang pharmaceutical Co., Ltd., with purity of 98%-102%;
crospolyvinylpyrrolidone: purchased from Chongqing Star-Tech & JRS Specialty Products Co., Ltd., under the trade name of Oridon^{®}XL;
polyethylene oxide: available from DUPONT, under the trade name of POLYOX^{™}; and KSR: available from BSAF, under the trade name of KOLLIDON^{®}SR, labeled as an 80/19 (w/w) mixture of PVAc and PVP.

### Example 1

Oval tablets A, B, C, D, E, F, G and H were prepared using a direct tableting method having the following tablet core composition (Tables 1-1 and 1-2).

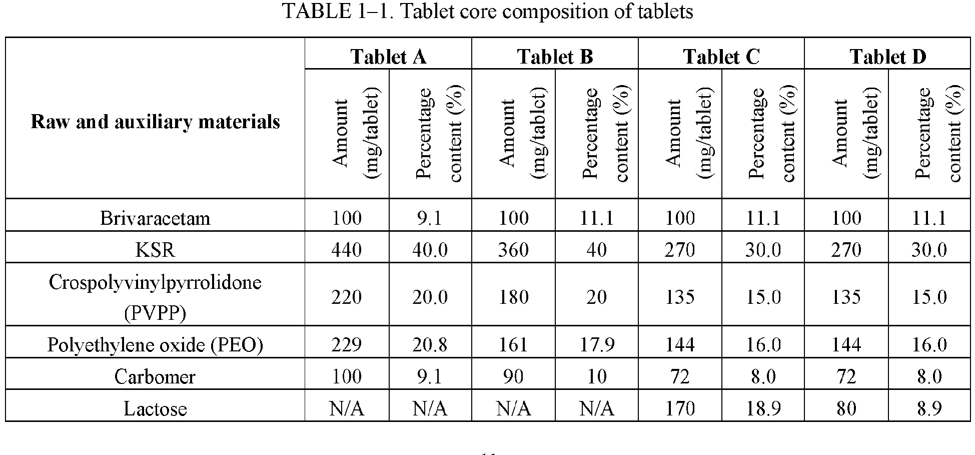

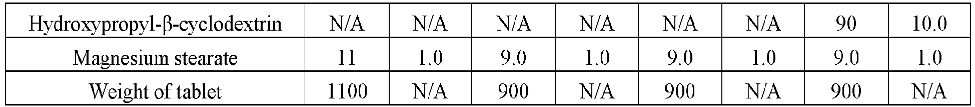

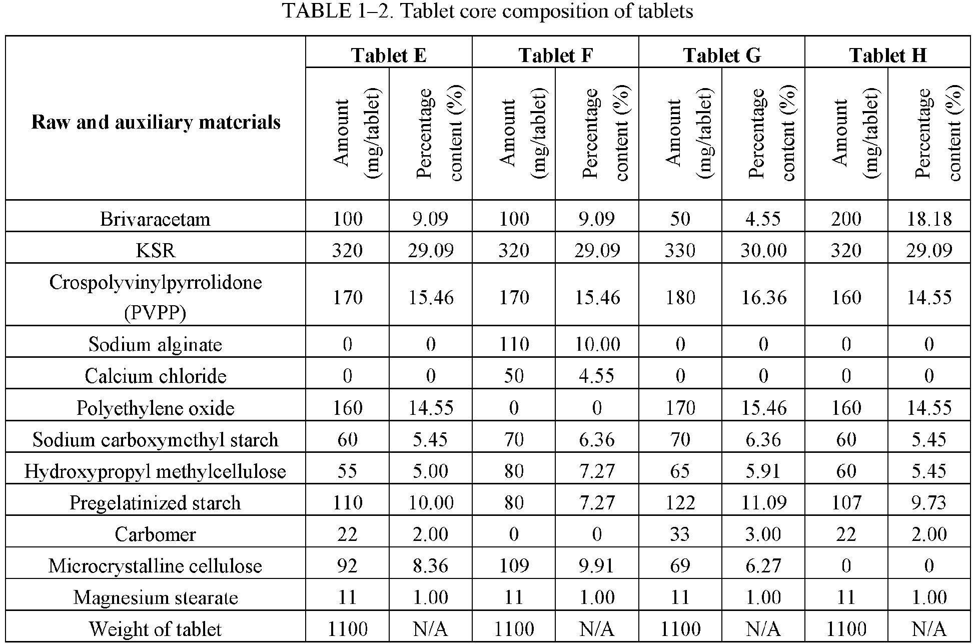

### (I) Dissolution test

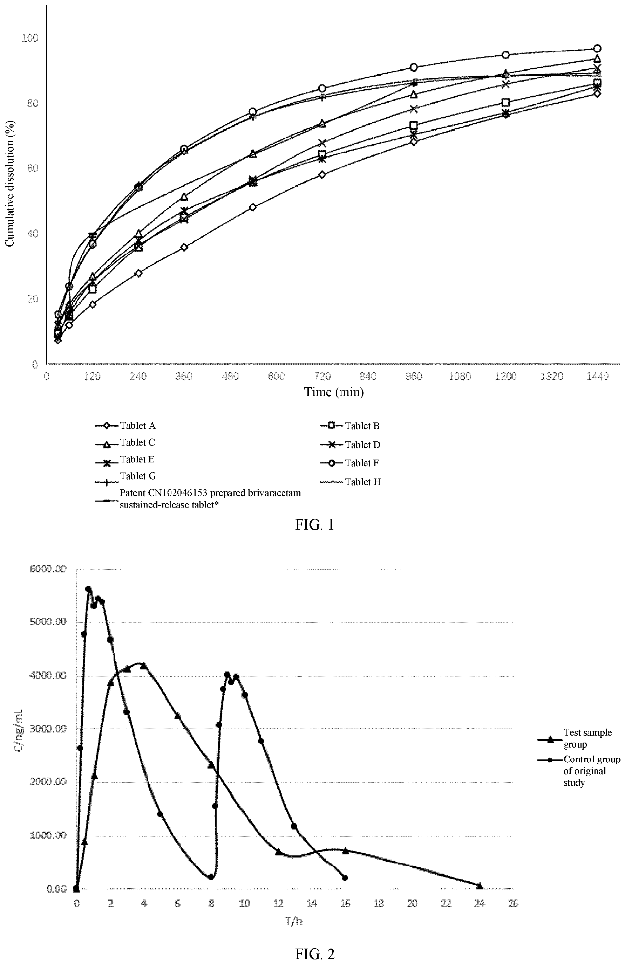

Dissolution results (Table 2) show that the *in vitro* dissolution of tablets A-H (acetate buffer with a pH of 4.5, according to the Method 2 of General Rules 0931 of Chinese Pharmacopoeia (2020 Version), 50 rpm, 37 ± 0.5 °C, 900 mL) meets the requirements. Compared with the example with the slowest release rate in patent CN102046153B (Table 2), the tablet samples prepared in Example 1 of the present disclosure dissolved out more slowly (see FIG. 1 for dissolution curve diagram). Compared with the dissolution results (Table 3) of the common tablet (commercially available brivaracetam tablet, available from UCB, Inc., under the trade name of BRIVIACT, prescription shown in Table A), the tablet samples prepared in Example 1 have a significant sustained-release effect.

**TABLE A. Prescription of common tablet**

| **Materials** | **Prescription of common tablet (specification: 50 mg)** | |
|---|---|---|
| | **mg/tablet** | **Weight percentage (%)** |
| Brivaracetam | 50 | 18.52 |
| Lactose monohydrate | 97 | 35.93 |
| Anhydrous lactose | 96.5 | 35.74 |
| B-cyclodextrin | 13.5 | 5.00 |
| Croscarmellose sodium | 10 | 3.70 |
| Magnesium stearate | 3 | 1.11 |
| Tablet core | 270 | 100.0 |
| Coating powder | 10.8 | 4.00 |

**TABLE 2. Dissolution results of different tablets**

| **Tablets** | **Time (min)** | **30** | **60** | **120** | **240** | **360** | **540** | **720** | **960** | **1200** | **1440** |
|---|---|---|---|---|---|---|---|---|---|---|---|
| Tablet A | Cumulative dissolution (%) | 7.5 | 11.9 | 18.4 | 28.0 | 35.9 | 48.1 | 58.1 | 68.3 | 76.4 | 83.0 |
| Tablet B | Cumulative dissolution (%) | 9.6 | 14.8 | 23.1 | 36 | 45.1 | 55.9 | 64.3 | 73.2 | 80.3 | 86.2 |
| Tablet C | Cumulative dissolution (%) | 12.1 | 18.4 | 27.1 | 40.2 | 51.5 | 64.7 | 73.9 | 82.8 | 89.2 | 93.7 |
| Tablet D | Cumulative dissolution (%) | 11.6 | 17.6 | 25.5 | 36.4 | 44.5 | 56.6 | 67.9 | 78.3 | 85.9 | 90.9 |
| Tablet E | Cumulative dissolution (%) | 8.3 | 15.6 | 25.4 | 37.9 | 47.0 | 55.9 | 63.1 | 70.4 | 77.1 | 85.2 |
| Tablet F | Cumulative dissolution (%) | 15.3 | 24.0 | 36.8 | 54.3 | 66.1 | 77.4 | 84.6 | 91.0 | 94.9 | 96.8 |
| Tablet G | Cumulative dissolution (%) | 13.1 | 23.6 | 39.1 | 54.9 | 65.2 | 75.8 | 81.7 | 86.3 | 88.4 | 89.3 |
| Tablet H | Cumulative dissolution (%) | 15.1 | 23.7 | 36.5 | 53.5 | 64.9 | 75.7 | 82.4 | 87.1 | 88.5 | 88.5 |
| Patent CN102046153B Example 1 prepared Brivaracetam sustained -release tablet C^{∗} | Cumulative dissolution (%) | N/A | 14 | 40 | N/A | N/A | N/A | N/A | 86 | N/A | N/A |

| | | | | | | | | | | | |
|---|---|---|---|---|---|---|---|---|---|---|---|
| Note: ^{∗} is the result of the slowest dissolution rate of the brivaracetam sustained-release tablet of the example disclosed in CN102046153B. | | | | | | | | | | | |

**TABLE 3. Dissolution results of common tablet (acetate buffer with a pH of 4.5)**

| **Tablet** | **Time (min)** | **5** | **10** | **15** | **20** | **30** |
|---|---|---|---|---|---|---|
| Common tablet | Cumulative dissolution (%) | 74.8 | 100.3 | 101.8 | 101.9 | 101.9 |

| | | | | | | |
|---|---|---|---|---|---|---|
| Note: the cumulative dissolution value exceeding 100% is caused by detection error. | | | | | | |

From the above dissolution rate data, the brivaracetam pharmaceutical composition/tablet disclosed herein has a dissolution rate of more than 83% in about 24 h, indicating that the sustained release effect is significantly superior to that of a common tablet and the brivaracetam sustained-release tablet disclosed in patent CN102046153B.

### (II) PK Study in Beagle dogs

In the test, 3 Beagle dogs comprising 2 males and 1 female dog were selected. Both the test sample group (tablet E in Example 1) and the control group of original study (i.e., common tablet) were administered at a dose (based on active ingredients) of 100 mg/dose/day, and the test sample group was administered at a single dose, and the control group of original study was administered 1 tablet per 8 h for 2 times. 3 Beagle dogs, orally administered the test sample, served as a test sample group, and blood samples were collected before the administration (one day before administration) and at time points of 0.5 h, 1 h, 2 h, 3 h, 4 h, 6 h, 8 h, 12 h, 16 h, 24 h, 30 h, 36 h and 48 h after the administration; after 7 days (the test sample was completely eluted), the 3 Beagle dogs, orally administered the original drug, served as a control group of original study, and blood samples were collected before the administration (one day before administration) and at time points of 0.25 h, 0.5 h, 0.75 h, 1 h, 1.25 h, 1.5 h, 2 h, 3 h, 5 h, 8 h (second administration after blood collection), 8.25 h, 8.5 h, 8.75 h, 9 h, 9.25 h, 9.5 h, 10 h, 11 h, 13 h, 16 h and 24 h.

The results are shown in Table 4.

**TABLE 4**

| Group | Parameters | t_{1/2} | Tmax | Cmax | AUC₀₋₂₄ₕ | AUC_{inf} | MRTₗₐₛₜ |
|---|---|---|---|---|---|---|---|
| | | h | h | ng/mL | h·ng/mL | h·ng/mL | h |
| Control group of original study | Mean | 1.33 | 0.83 | 6313.36 | 35925.93 | 36312.55 | 6.00 |
| | SD | 0.19 | 0.63 | 968.06 | 1189.23 | 1127.73 | 0.40 |
| Test sample group | Mean | 2.43 | 3.00 | 4552.41 | 37163.80 | 37507.03 | 6.19 |
| | SD | 1.95 | 1.00 | 413.58 | 8641.66 | 8584.45 | 1.45 |

The results show that the half-life period and the time to peak of the test sample group are prolonged, the maximum blood concentration is lower than that of the control group of original study (namely the commercially available product), and the AUC value is close to that of the control group of original study (see FIG. 2 for the time-concentration curve).

### (III) Stability test

The samples were placed under high temperature and high humidity conditions (60 °C/saturated potassium nitrate solution, with humidity of 75% RH) for 30 days, sampled on days 10 and 30, and tested for the relevant substances and dissolution curves, and the stability was examined as follows:

**TABLE 5**

| **Sample/Placement time** | | **Impurity B** | **Impurity M** | **Total impurities (except impurity M)** |
|---|---|---|---|---|
| Tablet A | Day 0 | 0.04% | Undetectable | 0.08% |
| | Day 10 | 0.04% | Undetectable | 0.08% |
| | Day 30 | 0.04% | 0.06% | 0.09% |
| Tablet E | Day 0 | 0.04% | Undetectable | 0.07% |
| | Day 10 | 0.04% | Undetectable | 0.08% |
| | Day 30 | 0.04% | 0.02% | 0.10% |
| Acceptance criteria | | ≤ 0.20% | ≤ 2.50% | ≤ 0.70% |

**TABLE 6**

| **Tablets** | **Placement time** | **Time (min)** | **30** | **60** | **120** | **240** | **360** | **540** | **720** | **960** | **1200** | **1440** |
|---|---|---|---|---|---|---|---|---|---|---|---|---|
| Tablet A | Day 0 | Cumulative dissolution (%) | 7.5 | 11.9 | 18.4 | 28.0 | 35.9 | 48.1 | 58.1 | 68.3 | 76.4 | 83.0 |
| | Day 10 | Cumulative dissolution (%) | 9.2 | 13.8 | 20.5 | 30.1 | 37.6 | 47.7 | 56.3 | 65.2 | 75.1 | 81.5 |
| | Day 30 | Cumulative dissolution (%) | 11.7 | 18.2 | 27.6 | 39.4 | 46.7 | 56.2 | 62.3 | 74.9 | 80.2 | 87.3 |
| Tablet E | Day 0 | Cumulative dissolution (%) | 8.3 | 15.6 | 25.4 | 37.9 | 47.0 | 55.9 | 63.1 | 70.4 | 77.1 | 85.2 |
| | Day 10 | Cumulative dissolution (%) | 11.3 | 18.9 | 29.5 | 41.6 | 49.4 | 57.7 | 64.3 | 71.0 | 76.3 | 84.6 |
| | Day 30 | Cumulative dissolution (%) | 10.6 | 17.0 | 26.1 | 37.8 | 45.7 | 54.5 | 61.4 | 69.0 | 74.5 | 83.0 |

The results show that the brivaracetam sustained-release tablet prepared herein has good stability.

The examples of the present disclosure have been described above. However, the present disclosure is not limited to the above examples. Any modification, equivalent, improvement and the like made without departing from the spirit and principle of the present disclosure shall fall within the protection scope of the present disclosure.

## Claims

1. A brivaracetam pharmaceutical composition, wherein the brivaracetam pharmaceutical composition is a 24-hour sustained-release drug and has a dissolution simultaneously meeting the following three characteristics:
A) not more than 40% of an active pharmaceutical ingredient is dissolved out within 1 h;
B) 20%-70% of the active pharmaceutical ingredient is dissolved out within 6 h; and
C) not less than 65% of the active pharmaceutical ingredient is dissolved out within 24 h;
the active pharmaceutical ingredient is selected from one, two or more of brivaracetam, a pharmaceutically acceptable complex of the brivaracetam, a pharmaceutically acceptable salt of the brivaracetam, a pharmaceutically acceptable solvate of the brivaracetam and a pharmaceutically acceptable hydrate of the brivaracetam;
preferably, the brivaracetam pharmaceutical composition has a dissolution simultaneously meeting the following three characteristics:
A) not more than 40% of the brivaracetam or the pharmaceutically acceptable salt thereof is dissolved out within 1 h;
B) 20%-70% of the brivaracetam or the pharmaceutically acceptable salt thereof is dissolved out within 6 h; and
C) not less than 65% of the brivaracetam or the pharmaceutically acceptable salt thereof is dissolved out within 24 h.

2. The brivaracetam pharmaceutical composition according to claim 1, wherein the brivaracetam pharmaceutical composition is a 24-hour sustained-release drug and has a dissolution simultaneously meeting the following three characteristics:
A) not more than 35% of the active pharmaceutical ingredient is dissolved out within 1 h; preferably, not more than 35% of the brivaracetam or the pharmaceutically acceptable salt thereof is dissolved out within 1 h;
B) 30%-70% of the active pharmaceutical ingredient is dissolved out within 6 h; preferably, 30%-70% of the brivaracetam or the pharmaceutically acceptable salt thereof is dissolved out within 6 h; and
C) not less than 75% of the active pharmaceutical ingredient is dissolved out within 24 h; preferably, not less than 75% of the brivaracetam or the pharmaceutically acceptable salt thereof is dissolved out within 24 h;
preferably, the brivaracetam pharmaceutical composition is a 24-hour sustained-release drug and has a dissolution simultaneously meeting the following three characteristics:
A) not more than 30% of the active pharmaceutical ingredient is dissolved out within 1 h; preferably, not more than 30% of the brivaracetam or the pharmaceutically acceptable salt thereof is dissolved out within 1 h;
B) 30%-65% of the active pharmaceutical ingredient is dissolved out within 6 h; preferably, 30%-65% of the brivaracetam or the pharmaceutically acceptable salt thereof is dissolved out within 6 h; and
C) not less than 75% of the active pharmaceutical ingredient is dissolved out within 24 h; preferably, not less than 75% of the brivaracetam or the pharmaceutically acceptable salt thereof is dissolved out within 24 h;
also preferably, the brivaracetam pharmaceutical composition is a 24-hour sustained-release drug and has a dissolution simultaneously meeting the following three characteristics:
A) not more than 30% of the active pharmaceutical ingredient is dissolved out within 1 h; preferably, not more than 30% of the brivaracetam or the pharmaceutically acceptable salt thereof is dissolved out within 1 h;
B) 30%-65% of the active pharmaceutical ingredient is dissolved out within 6 h; preferably, 30%-65% of the brivaracetam or the pharmaceutically acceptable salt thereof is dissolved out within 6 h; and
C) not less than 80% of the active pharmaceutical ingredient is dissolved out within 24 h; preferably, not less than 80% of the brivaracetam or the pharmaceutically acceptable salt thereof is dissolved out within 24 h.

3. A brivaracetam pharmaceutical composition, comprising an active pharmaceutical ingredient, a matrix forming agent and a swelling agent; wherein:
the active pharmaceutical ingredient is selected from one, two or more of the following substances:
brivaracetam, a pharmaceutically acceptable complex of the brivaracetam, a pharmaceutically acceptable salt of the brivaracetam, a pharmaceutically acceptable solvate of the brivaracetam, and a pharmaceutically acceptable hydrate of the brivaracetam;
the matrix forming agent is selected from one, two or more of the following substances: one or more of polyvinyl acetate, polyvinylpyrrolidone, KSR, carbomer, polysaccharide, polyacrylic resin, polyvinyl acetate povidone mixture, and polyvinyl alcohol;
the swelling agent is selected from one, two or more of the following substances: one or more of crospolyvinylpyrrolidone, polyethylene oxide, croscarmellose sodium, sodium carboxymethyl starch, low-substituted hydroxypropylcellulose, carboxymethylcellulose calcium, carboxymethylcellulose, alginate, calcium salt, and polacrilin potassium;
preferably, the active pharmaceutical ingredient is brivaracetam;
and/or,
the matrix forming agent is one, two or more of polyvinyl acetate, polyvinylpyrrolidone, KSR, carbomer and polysaccharide;
and/or,
the swelling agent is one, two or more of crospolyvinylpyrrolidone, polyethylene oxide and sodium carboxymethyl starch;
still preferably, a weight percentage of the active pharmaceutical ingredient is 2.00%-50.00%; wherein the weight percentage refers to the percentage of the weight of the active pharmaceutical ingredient in the total weight of the brivaracetam pharmaceutical composition;
and/or,
a weight percentage of the matrix forming agent is 5.00%-60.00%, wherein the weight percentage refers to the percentage of the weight of the matrix forming agent in the total weight of the brivaracetam pharmaceutical composition;
and/or,
a weight percentage of the swelling agent is 5.00%-60.00%, wherein the weight percentage refers to the percentage of the weight of the swelling agent in the total weight of the brivaracetam pharmaceutical composition;
preferably, a weight percentage of the active pharmaceutical ingredient is 3.00%-20.00%, wherein the weight percentage refers to the percentage of the weight of the active pharmaceutical ingredient in the total weight of the brivaracetam pharmaceutical composition;
and/or,
a weight percentage of the matrix forming agent is 30.00%-50.00%, wherein the weight percentage refers to the percentage of the weight of the matrix forming agent in the total weight of the brivaracetam pharmaceutical composition;
and/or,
a weight percentage of the swelling agent is 20.00%-50.00%, wherein the weight percentage refers to the percentage of the weight of the swelling agent in the total weight of the brivaracetam pharmaceutical composition.

4. The brivaracetam pharmaceutical composition according to claim 3, wherein the polysaccharide is selected from one, two or more of the following substances: xanthan gum, inulin, guar gum, chitosan, carob gum, carrageenan and a cellulose derivative;
preferably, the cellulose derivative is selected from one, two or more of ionic cellulose polymer and nonionic cellulose polymer;
preferably, the ionic cellulose polymer is selected from one, two or more of the following substances:
carboxymethylcellulose, carboxymethylcellulose sodium, carboxymethylcellulose calcium, carboxyethylcellulose, carboxymethylethylcellulose, hydroxyethyl methylcellulose acetate phthalate, hydroxyethyl methylcellulose acetate succinate, hydroxypropylcellulose acetate phthalate, hydroxypropylcellulose acetate succinate, and hydroxypropyl methylcellulose acetate phthalate;
and/or,
the nonionic cellulose polymer is selected from one, two or more of the following substances: methylcellulose, ethylcellulose, hydroxyethylcellulose, hydroxypropylcellulose, hydroxypropyl methylcellulose, hydroxypropyl methylcellulose acetate, hydroxyethyl methylcellulose, hydroxyethylcellulose acetate, and hydroxyethyl ethylcellulose.

5. The brivaracetam pharmaceutical composition according to claim 3 or 4, wherein the brivaracetam pharmaceutical composition further comprises a lubricant and/or a diluent;
preferably, the lubricant is selected from one, two or more of talc, stearic acid, metal stearate, stearate, glyceryl behenate, sodium lauryl sulfate, hydrogenated vegetable oil, mineral oil, poloxamer, polyethylene glycol and sodium chloride;
and/or,
the diluent is selected from one, two or more of dextrose, lactose monohydrate, anhydrous lactose, sucrose, mannitol, xylitol, sorbitol, microcrystalline cellulose, starch, pregelatinized starch, dicalcium phosphate dihydrate, anhydrous dicalcium phosphate, cyclodextrin and cyclodextrin derivative;
preferably, the metal stearate is selected from one, two or more of calcium stearate, magnesium stearate and zinc stearate;
and/or,
the stearate is selected from one, two or more of polyoxyethylene stearate, glyceryl monostearate and glyceryl palmitostearate;
and/or,
the cyclodextrin is selected from one, two or more of α-cyclodextrin, β-cyclodextrin and gamma-cyclodextrin;
and/or,
the cyclodextrin derivative is selected from one, two or more of cyclodextrin glucose derivative, cyclodextrin hydroxypropyl derivative, cyclodextrin methyl derivative, cyclodextrin ethyl derivative, cyclodextrin acetyl derivative, cyclodextrin sulfobutyl derivative and ionic cyclodextrin derivative;
preferably, a weight percentage of the lubricant is 0%-3.00%, wherein the weight percentage refers to the percentage of the weight of the lubricant in the total weight of the brivaracetam pharmaceutical composition;
and/or,
a weight percentage of the diluent is 0%-30.00%, wherein the weight percentage refers to the percentage of the weight of the diluent in the total weight of the brivaracetam pharmaceutical composition;
preferably, a weight percentage of the lubricant is 0.50%-2.00%, wherein the weight percentage refers to the percentage of the weight of the lubricant in the total weight of the brivaracetam pharmaceutical composition;
and/or,
a weight percentage of the diluent is 0%-20.00%, wherein the weight percentage refers to the percentage of the weight of the diluent in the total weight of the brivaracetam pharmaceutical composition.

6. The brivaracetam pharmaceutical composition according to any one of claims 3-5, wherein:
the brivaracetam pharmaceutical composition consists of the active pharmaceutical ingredient, the matrix forming agent and the swelling agent;
the active pharmaceutical ingredient is selected from one, two or more of brivaracetam, a pharmaceutically acceptable complex of the brivaracetam, a pharmaceutically acceptable salt of the brivaracetam, a pharmaceutically acceptable solvate of the brivaracetam and a pharmaceutically acceptable hydrate of the brivaracetam;
the matrix forming agent is selected from one, two or more of polyvinyl acetate, polyvinylpyrrolidone, KSR,
carbomer and polysaccharide;
the swelling agent is selected from one, two or more of crospolyvinylpyrrolidone, polyethylene oxide,
alginate and calcium salt;
preferably, the brivaracetam pharmaceutical composition consists of the following components:
brivaracetam, KSR, crospolyvinylpyrrolidone, polyethylene oxide, carbomer and magnesium stearate;
or,
the brivaracetam pharmaceutical composition consists of the following components: brivaracetam, KSR, crospolyvinylpyrrolidone, polyethylene oxide, carbomer, lactose and magnesium stearate;
or,
the brivaracetam pharmaceutical composition consists of the following components: brivaracetam, KSR, crospolyvinylpyrrolidone, polyethylene oxide, sodium carboxymethyl starch, hydroxypropyl methylcellulose, pregelatinized starch, carbomer, microcrystalline cellulose and magnesium stearate;
or,
the brivaracetam pharmaceutical composition consists of the following components: brivaracetam, KSR, crospolyvinylpyrrolidone, sodium alginate, calcium chloride, sodium carboxymethyl starch, hydroxypropyl methylcellulose, pregelatinized starch, microcrystalline cellulose and magnesium stearate;
or,
the brivaracetam pharmaceutical composition consists of the following components: brivaracetam, KSR, crospolyvinylpyrrolidone, sodium carboxymethyl starch, hydroxypropyl methylcellulose, pregelatinized starch, carbomer, microcrystalline cellulose and magnesium stearate;
or,
the brivaracetam pharmaceutical composition consists of the following components: brivaracetam, KSR, crospolyvinylpyrrolidone, polyethylene oxide, sodium carboxymethyl starch, hydroxypropyl methylcellulose, pregelatinized starch, carbomer and magnesium stearate;
preferably, the brivaracetam pharmaceutical composition is selected from any one of the following formulations:
formulation I: 9.1% of brivaracetam, 40.0% of KSR, 20.0% of crospolyvinylpyrrolidone, 20.8% of polyethylene oxide, 9.1% of carbomer, and 1.0% of magnesium stearate; the percentage refers to a weight percentage, wherein the weight percentage refers to the percentage of the weight of each component in the total weight of the pharmaceutical composition;
formulation II: 11.1% of brivaracetam, 40.0% of KSR, 20.0% of crospolyvinylpyrrolidone, 17.9% of polyethylene oxide, 10.0% of carbomer, and 1.0% of magnesium stearate; the percentage refers to a weight percentage, wherein the weight percentage refers to the percentage of the weight of each component in the total weight of the brivaracetam pharmaceutical composition;
formulation III: 11.1% of brivaracetam, 30.0% of KSR, 15.0% of crospolyvinylpyrrolidone, 16.0% of polyethylene oxide, 8.0% of carbomer, 18.9% of lactose, and 1.0% of magnesium stearate; the percentage refers to a weight percentage, wherein the weight percentage refers to the percentage of the weight of each component in the total weight of the brivaracetam pharmaceutical composition;
formulation IV: 11.1% of brivaracetam, 30.0% of KSR, 15.0% of crospolyvinylpyrrolidone, 16.0% of polyethylene oxide, 8.0% of carbomer, 8.9% of lactose, 10.0% of hydroxypropyl-β-cyclodextrin, and 1.0% of magnesium stearate; the percentage refers to a weight percentage, wherein the weight percentage refers to the percentage of the weight of each component in the total weight of the brivaracetam pharmaceutical composition;
formulation V: 9.09% of brivaracetam, 29.09% of KSR, 15.46% of crospolyvinylpyrrolidone, 14.55% of polyethylene oxide, 5.45% of sodium carboxymethyl starch, 5.00% of hydroxypropyl methylcellulose, 10.00% of pregelatinized starch, 2.0% of carbomer, 8.36% of microcrystalline cellulose, and 1.0% of magnesium stearate; the percentage refers to a weight percentage, wherein the weight percentage refers to the percentage of the weight of each component in the total weight of the brivaracetam pharmaceutical composition;
formulation VI: 9.09% of brivaracetam, 29.09% of KSR, 15.46% of crospolyvinylpyrrolidone, 10.0% of sodium alginate, 4.55% of calcium chloride, 6.36% of sodium carboxymethyl starch, 7.27% of hydroxypropyl methylcellulose, 7.27% of pregelatinized starch, 9.91% of microcrystalline cellulose, and 1.0% of magnesium stearate; the percentage refers to a weight percentage, wherein the weight percentage refers to the percentage of the weight of each component in the total weight of the brivaracetam pharmaceutical composition;
formulation VII: 4.55% of brivaracetam, 30.0% of KSR, 16.36% of crospolyvinylpyrrolidone, 15.46% of polyethylene oxide, 6.36% of sodium carboxymethyl starch, 5.91% of hydroxypropyl methylcellulose, 11.09% of pregelatinized starch, 3.0% of carbomer, 6.27% of microcrystalline cellulose, and 1.0% of magnesium stearate; the percentage refers to a weight percentage, wherein the weight percentage refers to the percentage of the weight of each component in the total weight of the brivaracetam pharmaceutical composition; and
formulation VIII: 18.18% of brivaracetam, 29.09% of KSR, 14.55% of crospolyvinylpyrrolidone, 14.55% of polyethylene oxide, 5.45% of sodium carboxymethyl starch, 5.45% of hydroxypropyl methylcellulose, 9.73% of pregelatinized starch, 2.0% of carbomer, and 1.0% of magnesium stearate; the percentage refers to a weight percentage, wherein the weight percentage refers to the percentage of the weight of each component in the total weight of the brivaracetam pharmaceutical composition;
preferably, the brivaracetam pharmaceutical composition has the dissolution characteristics according to claim 1 or 2.

7. A brivaracetam sustained-release tablet, consisting of a tablet core and a coating, wherein the tablet core comprises the pharmaceutical composition according to any one of claims 1-6;
preferably, the tablet core consists of the following components in percentage by weight: 2.00%-50.00% of the brivaracetam, 5.00%-60.00% of the matrix forming agent, 5.00%-60.00% of the swelling agent, 0%-3.00% of the lubricant and 0%-30.00% of the diluent, wherein the weight percentage refers to the percentage of the weight of each component in the total weight of the brivaracetam sustained-release tablet;
preferably, the tablet core of the brivaracetam sustained-release tablet consists of the following components in percentage by weight: 3.00%-20.00% of the brivaracetam, 30.00%-50.00% of the matrix forming agent, 20.00%-50.00% of the swelling agent, 0.50%-2.00% of the lubricant and 0%-20.00% of the diluent, wherein the weight percentage refers to the percentage of the weight of each component in the total weight of the brivaracetam sustained-release tablet.

8. The brivaracetam sustained-release tablet according to claim 7, wherein:
the tablet core of the brivaracetam sustained-release tablet is any one of the following prescriptions:
prescription I: 9.1% of brivaracetam, 40.0% of KSR, 20.0% of crospolyvinylpyrrolidone, 20.8% of polyethylene oxide, 9.1% of carbomer, and 1.0% of magnesium stearate; the percentage refers to a weight percentage, wherein the weight percentage refers to the percentage of the weight of each component in the total weight of the tablet core of the brivaracetam sustained-release tablet;
prescription II: 11.1% of brivaracetam, 40.0% of KSR, 20.0% of crospolyvinylpyrrolidone, 17.9% of polyethylene oxide, 10.0% of carbomer, and 1.0% of magnesium stearate; the percentage refers to a weight percentage, wherein the weight percentage refers to the percentage of the weight of each component in the total weight of the tablet core of the brivaracetam sustained-release tablet;
prescription III: 11.1% of brivaracetam, 30.0% of KSR, 15.0% of crospolyvinylpyrrolidone, 16.0% of polyethylene oxide, 8.0% of carbomer, 18.9% of lactose, and 1.0% of magnesium stearate; the percentage refers to a weight percentage, wherein the weight percentage refers to the percentage of the weight of each component in the total weight of the tablet core of the brivaracetam sustained-release tablet;
prescription IV: 11.1% of brivaracetam, 30.0% of KSR, 15.0% of crospolyvinylpyrrolidone, 16.0% of polyethylene oxide, 8.0% of carbomer, 8.9% of lactose, 10.0% of hydroxypropyl-β-cyclodextrin, and 1.0% of magnesium stearate; the percentage refers to a weight percentage, wherein the weight percentage refers to the percentage of the weight of each component in the total weight of the tablet core of the brivaracetam sustained-release tablet;
prescription V: 9.09% of brivaracetam, 29.09% of KSR, 15.46% of crospolyvinylpyrrolidone, 14.55% of polyethylene oxide, 5.45% of sodium carboxymethyl starch, 5.00% of hydroxypropyl methylcellulose, 10.00% of pregelatinized starch, 2.0% of carbomer, 8.36% of microcrystalline cellulose, and 1.00% of magnesium stearate; the percentage refers to a weight percentage, wherein the weight percentage refers to the percentage of the weight of each component in the total weight of the tablet core of the brivaracetam sustained-release tablet;
prescription VI: 9.09% of brivaracetam, 29.09% of KSR, 15.46% of crospolyvinylpyrrolidone, 10.00% of sodium alginate, 4.55% of calcium chloride, 6.36% of sodium carboxymethyl starch, 7.27% of hydroxypropyl methylcellulose, 7.27% of pregelatinized starch, 9.91% of microcrystalline cellulose, and 1.00% of magnesium stearate; the percentage refers to a weight percentage, wherein the weight percentage refers to the percentage of the weight of each component in the total weight of the tablet core of the brivaracetam sustained-release tablet;
prescription VII: 4.55% of brivaracetam, 30.0% of KSR, 16.36% of crospolyvinylpyrrolidone, 15.46% of polyethylene oxide, 6.36% of sodium carboxymethyl starch, 5.91% of hydroxypropyl methylcellulose, 11.09% of pregelatinized starch, 3.00% of carbomer, 6.27% of microcrystalline cellulose, and 1.00% of magnesium stearate; the percentage refers to a weight percentage, wherein the weight percentage refers to the percentage of the weight of each component in the total weight of the tablet core of the brivaracetam sustained-release tablet; and
prescription VIII: 18.18% of brivaracetam, 29.09% of KSR, 14.55% of crospolyvinylpyrrolidone, 14.55% of polyethylene oxide, 5.45% of sodium carboxymethyl starch, 5.45% of hydroxypropyl methylcellulose, 9.73% of pregelatinized starch, 2.00% of carbomer, and 1.00% of magnesium stearate; the percentage refers to a weight percentage, wherein the weight percentage refers to the percentage of the weight of each component in the total weight of the tablet core of the brivaracetam sustained-release tablet.

9. A preparation method for the brivaracetam pharmaceutical composition according to any one of claims 1-6 or the brivaracetam sustained-release tablet according to claim 7 or 8, wherein the preparation method is direct tableting, dry granulation, wet granulation or melt granulation.

10. Use of the brivaracetam pharmaceutical composition according to any one of claims 1-6 or the brivaracetam sustained-release tablet according to claim 7 or 8 for preparing a medicament;
preferably, the medicament is used for the treatment and/or prevention of the following diseases: epilepsy, Parkinson, dyskinesia, migraine, tremor, essential tremor, bipolar disorder, chronic disease, neuropathic pain or bronchial, asthma or allergic diseases, etc.
